(19) Europäisches Patentamt
European Patent Office
Office européen des brevets

(11) Publication number: **0 010 851**
**B1**

(12) # EUROPEAN PATENT SPECIFICATION

(45) Date of publication of patent specification: **04.08.82**

(21) Application number: **79301967.0**

(22) Date of filing: **21.09.79**

(51) Int. Cl.³: **C 07 D 513/04,**
C 07 C 93/04,
C 07 C 103/44,
C 07 C 103/82,
C 07 D 233/42
//(C07D513/04, 277/00, 235/00)

(54) **Process for the preparation of tetramisole.**

(30) Priority: **06.11.78 US 958216**
**06.11.78 US 958222**
**02.08.79 US 63289**

(43) Date of publication of application:
**14.05.80 Bulletin 80/10**

(45) Publication of the grant of the patent:
**04.08.82 Bulletin 82/31**

(84) Designated Contracting States:
**AT BE CH DE FR GB IT LU NL SE**

(56) References cited:
**DE - A - 2 829 822**
**FR - A - 2 258 379**
**US - A - 3 726 894**
**US - A - 3 845 070**
**US - A - 3 873 560**
**US - A - 4 090 025**

(73) Proprietor: **AMERICAN CYANAMID COMPANY**
**Berdan Avenue**
**Wayne New Jersey 06904 (US)**

(72) Inventor: **Raghu, Sivaraman**
**45 Lois Street**
**Norwalk, Connecticut (US)**
Inventor: **Kurose, Nancy S.**
**10 Wayfaring Road**
**Norwalk, Connecticut (US)**

(74) Representative: **Allam, Peter Clerk et al,**
**LLOYD WISE, TREGEAR & CO. Norman House**
**105-109 Strand**
**London WC2R 0AE (GB)**

**EP 0 010 851 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of tetramisole

The present invention relates to a process for the preparation of tetramisole.

Certain imidazothiazoles have been found to have useful pharmaceutical and veterinary activity. For instance, the synthesis of tetramisole, or racemic 2,3,5,6-tetrahydro-6-phenylimidazo[2,1-b]thiazole, and its pharmaceutically acceptable addition salts is of considerable commercial interest because of the anthelminthic activity of such compounds. The enantiomers of this compound are well known and the laevorotatory is extremely well suited to such uses, as discussed in U.S. Patent 4,463,786.

As a consequence of such activity, various syntheses are known. In this connection, there are cited Raeymaekers et al., *J. Med. Chem. 9*, 545 (1966); Bakelien et al., *Aust. J. Chem. 21*, 1557 (1968); Roy U.S. Patent 3,855,234; McMenim U.S. Patent 3,845,070; and Spicer U.S. Patent 3,726,894.

Briefly, the process for the preparation of tetramisole in accordance with the present invention comprise reacting an arylvinyl oxide with an alkoxyethylamine to produce N-substituted alkoxyethylamines. The N-substituted alkoxyethylamines are thereafter reacted with a nitrile to provide an amidoamine which is then hydrolyzed to obtain a diamine. The diamine so produced is reacted with carbon disulfide to form a dithiocarbonate which is thereafter heated to form a thione which is then reacted with specific acids to provide a tetramisole salt. The tetramisole itself is thereafter obtained, if desired, by hydrolysis with a base.

The arylvinyl oxide starting material used in the practice of the present invention has the general formula

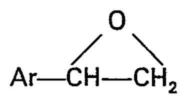

(I).

Ar represents phenyl.

The arylvinyl oxide is reacted with an alkoxyethylamine having the formula $H_2N—CH_2—CH_2—OR_1$ (II), where $R_1$ is a ($C_1$—$C_6$) alkyl group, for example methyl, ethyl, propyl, butyl, isopropyl, isobutyl, amyl, and hexyl. In certain embodiments, methoxyethylamine is especially preferred as reactant (II).

The reaction of the arylvinyl oxide and the alkoxyethylamine provides N-substituted alkoxyethylamines in high yields and with surprising regioselectivity. These N-substituted alkoxyethylamines have the formula $Ar—CH(OH)—CH_2—NH—CH_2CH_2—OR_1$ (III), wherein Ar and $R_1$ have the meaning set forth herein. A particularly preferred compound is one in which $R_1$ is methyl or ethyl.

The reaction of the arylvinyl oxide and the alkoxyethylamine can be conducted with or without an inert reaction vehicle. The reaction vehicle can serve to facilitate the mixing of the reactants, moderate the course of the reaction, and improve thermal control of the reaction. Suitable reaction vehicles include hydrocarbons and halogenated hydrocarbons. The reaction vehicles are chosen with a view toward maintaining the particular temperature and pressure conditions desired in the reaction so that undue volatility is avoided, while recovery of the product is facilitated. The hydrocarbons can include aliphatic, cycloaliphatic, and aromatic hydrocarbons. The desirable hydrocarbons are saturated aliphatic hydrocarbons having from about five to about 12 carbon atoms mononuclear and substituted mononuclear cycloaliphatics such as cyclohexane, cyclooctane, methylcyclohexane, and the like; and mononuclear aryl hydrocarbons and alkyl- and nitro- substituted mono- and polynuclear aryl hydrocarbons, such as benzene, toluene, xylene, chlorobenzene, and the like. The halogenated hydrocarbons contemplated for use in certain embodiments include lower halogenated hydrocarbons containing from one to four carbon atoms, such as methylene chloride, carbon tetrachloride, ethylene dichloride, and the like. Mixtures of the above-mentioned solvents may also be used. It has also been found very useful in certain embodiments of the invention to utilize an excess of alkoxyethylamine as the reaction vehicle.

The quantity of alkoxyethylamine is desirably at least sufficient on a molar basis to react with all of the arylvinyl oxide; that is to say, the alkoxyethylamine is used in amounts which are at least stoichiometric. When the alkoxyethylamine is used as a reaction vehicle, it can be present in substantial molar excess. The use of very large molar excesses creates the need to remove extra quantities of the starting alkoxyethylamine, although it is desirable to have sufficient to provide good reaction completeness. Thus, the quantity of alkoxyethylamine is from about one to about 15 times the molar quantity of the arylvinyl oxide, and in certain preferred embodiments, it is from about five to about ten molar quantities.

The reaction can be carried out by adding the arylvinyl oxide to the excess alkoxyethylamine. The reaction can be carried out over a wide temperature range, depending upon the reaction velocity with the particular reactants and upon the pressures utilized. Generally, the temperature is controlled to provide a smooth reaction during the initial contact of the reactants, and subsequently, if desired, the temperature can be raised to ensure good reaction completeness. The temperatures utilized can be

from 0° to 150°C, and preferred reaction temperatures are in the range of from 50° to 100°C. In certain preferred embodiments, the reaction is carried out under reflux of the alkoxyethylamine.

The reaction can be carried out over a range of pressures from subatmospheric to superatmospheric. Generally, no advantage accrues through the use of subatmospheric pressures, and it is accordingly desirable to use atmospheric or superatmospheric pressures up to three atmospheres. In certain particularly preferred embodiments, the reaction is carried out at reflux under atmospheric pressure.

The N-substituted alkoxyethylamine thus produced, directly or after recovery, is next reacted with a nitrile having the formula $R_2—C{\equiv}N$ (IV), where $R_2$ is hydrogen, alkyl or aryl. The desirable aryl groups are phenyl or substituted phenyl including lower alkyl mono- and polysubstituted phenyl, mono- and polyhalo phenyl, and the like. A preferred nitrile is benzonitrile.

The aliphatic nitrile is desirably a lower alkyl nitrile containing from two to about seven carbon atoms per molecule. The use of longer chain or unsaturated nitriles can complicate the process and increase the cost of the raw materials without any concomitant benefit. It is especially desirable to utilize the lower nitriles, such as acetonitrile and, as taught above, benzonitrile in certain preferred embodiments of the invention.

This reaction is carried out in the presence of a protic source with a reaction vehicle which is desirably aqueous. The requisite hydrogen ions are furnished through the use of a relatively strong aqueous acid. It is desirable to utilize an aqueous solution of a strong mineral acid, such as sulfuric acid, hydrochloric acid, and the like. The concentration of the mineral acid can range from about 50% by weight to the concentrated acid. Thus, a preferred protic source is concentrated sulfuric acid.

The reaction can be carried out with amounts of the nitrile ranging from equimolar, based upon the N-substituted ethylamine, up to an excess of ten times. When the nitrile is used in excess, it serves as a vehicle for the reaction, with attendant benefits in moderating and controlling the course of the reaction.

The reaction can be carried out at temperatures which provide reasonable reaction rates, while at the same time permitting control of the reaction velocity. In certain embodiments of the invention, it is desirable to use a temperature of from −25° to 60°C. The acid can be added to the nitrile at a lower temperature, and after acid addition is complete, the N-substituted ethylamine is slowly added to the nitrile and acid, desirably at a slow rate with good agitation. The addition of the amine is preferably carried out at temperatures up to 10°C, and after addition is complete, the temperature can be allowed to rise to room temperature (say, 18°—22°C) or higher, depending upon the particular reactants.

The reaction can be conducted over a wide range of pressures from subatmospheric to superatmospheric, and it has generally been found desirable to utilize atmospheric pressure. The reaction can also be effected in the presence of an inert reaction vehicle, such as those described for the preparation of the N-substituted alkoxyethylamine itself.

This process for reacting the N-substituted alkoxyethylamine and the nitrile provides N-(acylamino)alkoxyethylamines having the formula

$$R_2—CO—NH—CH(Ar)—CH_2—NH—CH_2CH_2—OR_1 \quad (V)$$

wherein Ar, $R_1$ and $R_2$ have the meanings set forth above. At this point amidoamine V can be obtained by neutralization, extraction with a suitable solvent, and solvent evaporation or other conventional recovery techniques. Alternatively it can also be used in the next step of the process without recovery.

In this next step the amidoamine V is hydrolysed to obtain a diamine having the formula

$$NH_2—CH(Ar)—CH_2—NH—CH_2CH_2—OR_1 \quad (VI)$$

wherein Ar and $R_1$ have the meaning set forth herein. The hydrolysis reaction is effected with a base or a protic source. The desirable protic sources are strong acids, such as mineral acids, whilst suitable bases are exemplified by the alkali metal hydroxides. Sulfuric acid is a preferred mineral acid, and sodium and potassium hydroxides are preferred bases. These protic sources or bases are desirably used in an aqueous milieu. Thus, a 10 to 50% aqueous sulfuric acid and a 5 to 50% alkali metal hydroxide have been found to provide good results in practicing this process.

The hydrolysis can be carried out over a range of temperatures from 0° to about 110°C. Generally, an initial reaction at a higher temperature, followed by a lower temperature over a longer period of time provides desirable reaction completeness. Accordingly, refluxing with the aqueous protic source or base for from one to six hours, followed by further contact at 20°—30°C for from 8—24 hours, provides good reaction completeness.

This reaction can be carried out over a range of pressures from subatmospheric to superatmospheric. Generally, no advantage is obtained in this process for hydrolysis by operating at subatmospheric pressures, while in some cases the reaction can be accelerated by operation at superatmospheric pressures. It is generally found, however, that atmospheric pressure provides adequate velocity and completeness, and such pressure is preferred.

The diamine VI and the processes for producing it, together with its predecessor intermediates,

are the key to the regioselective synthesis of tetramisole in accordance with the present invention. In the synthesis the diamine VI is reacted with carbon disulfide to produce a dithiocarbamate intermediate which can be represented by the tautomer

$$^{\ominus}SC(S)—NH—C(Ar)—CH_2—^{\oplus}NH_2—CH_2CH_2—OR_1 \quad (VII)$$

which is then cyclized with heat to produce 1-substituted-4-arylimidazolidin-2-thione having the formula

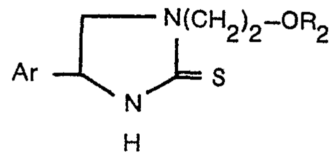

(VIII)

The thione so produced is then treated with an acid having a pharmaceutically acceptable anion to provide imidazothiazole:

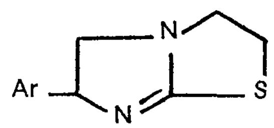

HA

(IX)

It will be recognized that these are the pharmaceutically acceptable salts of *d,1*-6-phenyl-2,3,5,6-tetrahydroimidazo[2,1-b]thiazole, or tetramisole. Such acid compounds can be neutralized with a base to provide the free tetramisole (X), when this is desired.

The dithiocarbamate is prepared by reacting diamine (VI) with carbon disulfide at temperatures of from —10° to 40°C. It is generally desirable to use from a 50 to 100% stoichiometric excess of carbon disulfide. This reaction step is desirably carried out in the presence of an inert vehicle such as one or more hydrocarbons or chlorinated hydrocarbons. Preferred hydrocarbons include lower alkyl, cycloalkyl, and aromatic materials such as benzene, toluene, xylene, and the like; liquid aliphatic hydrocarbons having five to 12 carbon atoms such as hexane, isooctane, heptane and the like; and cycloaliphatic materials such as cyclohexane, cyclooctane, and the like. The chlorinated hydrocarbons include the polyhalogenated lower aliphatic materials, a preferred vehicle being tetrachloroethane.

The reaction time ranges from about 30 minutes to about four hours in certain desirable embodiments of the invention. The resulting dithio compound VII is cyclized by heating at 80° to 150°C. The ring closure to provide thione VIII is carried out for from about two to about 20 hours. Production of pharmaceutically acceptable salt of the tetramisole is then effected on the thione by acid treatment to close the thiazolidine ring.

It will be understood from the present disclosure that the various intermediates can be recovered and purified as desired by conventional techniques such as extraction, solvent evaporation, water washing, and combinations of these conventional procedures. Further, the various steps can be carried out under subatmospheric or superatmospheric pressure. Unless superatmospheric pressure is desirable because of the volatility of a solvent or reactant, it is generally preferred to conduct all of the steps under atmospheric pressure. This provides further economy in not requiring special pressure vessels and handling techniques in commercial production.

## Example 1

Preparation of N-(2-Hydroxy-2-phenylethyl)-2-methoxyethylamine

To 157 g of refluxing 2-methoxyethylamine is added dropwise 25 g styrene oxide during a period of 15 minutes. After additon of the amine, the mixture is allowed to reflux for another two hours.

The reflux condenser is then replaced by a distillation head and the excess 2-methoxyethylamine is distilled off at atmospheric pressure. The last traces of 2-methoxyethylamine are distilled off under reduced pressure, and the residual solid is triturated with 100 ml of hexane.

The resulting material is filtered to provide the above-designated 2-methoxyethylamine derivative with a melting point of 68—70°C. IR (infrared) and NMR (proton nuclear magnetic resonance) spectroscopy confirm the structure.

## Example 2

Preparation of N-(2-Acetylamino-2-phenethyl)-2-methoxyethylamine

A flask is charged with 12.3 g (0.3 moles) of acetonitrile which is cooled to 0°C and maintained at a temperature of from 0° to 5°C, utilizing a bath temperature of —10° to —20°C. Thereafter, 80 g (0.82 moles) of concentrated sulfuric acid is added dropwise, and 19.5 g (0.1 mole) of the N-substituted hydroxyethylamine produced in Example 1 is added in small portions with efficient magnetic stirring so as to maintain the temperature between 0° and 10°C. The addition consumes about 30 to 35 minutes.

The reaction mixture is then stirred at 0°C for one hour and at 25°C for one and a half hours.

Thereafter, the reaction mixture is poured over 50 g of ice, and the resulting solution is added dropwise to 100 g of sodium hydroxide in 300 ml of water, while cooling in an ice bath with efficient magnetic stirring. The solution is extracted twice with 125 ml portions of methylene chloride. The two extracts are washed with water and dried over sodium sulfate.

Example 3

Preparation of N-(2-Amino-2-phenethyl)-2-methoxyethylamine

A flask is charged with a total of 23.6 g (0.1 mole) of the amidoamine produced in Example 2 and refluxed with 150 g of 20% aqueous sulfuric acid for three hours. Following the reflux, the reaction mixture is stirred at room temperature of about 22°C for an overnight period of 16 hours. The mixture is then neutralized to about pH 10 with cooling, utilizing sufficient 40% aqueous sodium hydroxide.

The reaction mixture is thrice extracted with 75 ml portions of methylene chloride. The extracts are then washed with water and dried over sodium sulfate. The solvent is stripped off to provide about 18.0 g of yellow oil. The IR is consistent with the desired diamine. The yellow oil is then distilled under reduced pressure.

The distillation produces 16.8 g of a liquid with a boiling point of 130°—135°C at 1 mm Hg.

Example 4

A flask is charged with 37.2 g (0.90 mole) of acetonitrile and cooled to 0°C, whereupon 100 ml (180 g, or 1.84 moles) of concentrates sulfuric acid is added dropwise while the temperature is maintained between 0° and 10°C utilizing a —10° to 0°C bath. After completion of the acid addition, 58.5 g (0.3 mole) of 2-phenyl-2-hydroxy-N-(2-methoxyethyl)ethylamine is added in portions over 35 minutes, while the temperature is still maintained between 0° and 10°C with the aforesaid cooling bath. Following addition of the amine, the mixture is stirred at 0° to 25°C for 90 minutes and then added to 300 ml of water. The aqueous material is refluxed for four hours, cooled, and stirred overnight at room temperature.

The cooled material is then neutralized to a pH of about 10 utilizing sufficient 40% aqueous sodium hydroxide and cooling. The neutralized product is extracted thrice with 200 ml quantities of methylene chloride. The methylene chloride extracts are washed and dried over sodium sulfate and concentrated to provide 53.6 g of oil. The IR spectrum indicates that a diamine is obtained.

The oil is distilled under vacuum to provide 48.2 g of colorless liquid 2-amino-2-phenyl-N-(2-methoxyethyl)ethylamine having a boiling point of 110°—115°C at 0.1—0.2 mm Hg. This represents an 82.8% yield, based upon the hydroxyethylamine starting material.

Example 5

Preparation of 2-Amino-2-phenyl-N-(2-methoxyethyl)ethylamine

A flask is charged with 43 g of benzonitrile and cooled to 0°C, whereupon 100 g of concentrated aqueous sulfuric acid is added dropwise, while the temperature is maintained between 0° and 10°C utilizing a —10° to 0°C cooling bath. After completion of the acid addition, 25 g of 2-phenyl-2-hydroxy-N-(2-methoxyethyl)ethylamine is added in portions over 45 minutes while the temperature is still maintained between 0° and 10°C with the aforesaid cooling bath. Following addition of the amine, the mixture is stirred at 0° to 25°C for 90 minutes and then added to 400 ml of water. The unreacted benzonitrile is extracted twice with 200 ml portions of methylene chloride and the aqueous material is refluxed for 24 hours.

The cooled material is thereupon neutralized to a pH of about 10 with 40% aqueous sodium hydroxide, while cooling. The neutralized product is extracted thrice with 100 ml quantities of methylene chloride. The methylene chloride extracts are washed, dried over sodium sulfate, and concentrated to provide 10 g of oil. The oil is distilled under vacuum to provide 13 g of colorless liquid 2-amino-2-phenyl-N-(2-methoxyethyl)ethylamine having a boiling point of 110°—115°C at 0.1—0.2 mm Hg.

Example 6

Preparation of 1-(2-Methoxyethyl)-4-phenylimidazolidin-2-thione

A flask is charged with 6.85 g of N-(2-amino-2-phenethyl)-2-methoxyethylamine in 20 ml of xylene, and this material is stirred with 3 ml of cabron disulfide at room temperature for two hours. The resulting slurry is then heated to 130°C and maintained at this temperature for four hours. The xylene is then distilled off under reduced pressure, and the thione product is identified by IR and NMR spectroscopic methods.

Example 7

Preparation of D,L-Tetramisole

A flask is charged with 4.3 of 1-(2-hydroxyethyl)-4-phenylimidazolidin-2-thione suspended in 50 ml of concentrated hydrochloric acid. The mixture is slowly heated to 70°C with magnetic stirring and maintained at this temperature for 10 hours. Thereafter, the flask contents are cooled and stirred at room temperature overnight.

The resulting solution is diluted with 50 ml of water, and the impurities are extracted with two 30 ml portions of methylene chloride. The aqueous layer is rendered basic with ammonium hydroxide and then extracted with three 50 ml portions of methylene chloride. The methylene chloride extracts are washed and dried. The solvent is evaporated to provide an oil which crystallizes. This oil is identified as (±)-6-phenyl-2,3,5,6-tetrahydroimidazo[2,1-b]thiazole by IR and NMR spectroscopy.

## Claims

1. A process for the preparation of tetramisole which comprises the steps of:

(a) reacting an arylvinyl oxide having the formula:

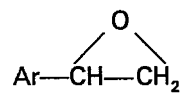

with an alkoxyethylamine having the formula: $R_1—O—(CH_2)_2—NH_2$ to obtain an N-(arylhydroxyalkyl)alkoxyethylamine having the formula: $Ar—CH(OH)—CH_2—NH—CH_2CH_2—OR_1$, wherein Ar is phenyl and $R_1$ is a ($C_1—C_6$) alkyl group,

(b) reacting the latter N-substituted alkoxyethylamine in the presence of a mineral acid with a nitrile having the formula $R_2C{\equiv}N$, where $R_2$ is hydrogen, alkyl, or aryl to obtain an amidoamine having the formula: $R_2—CO—NH—CH(Ar)—CH_2—NH—CH_2CH_2—OR_1$, where Ar, $R_1$ and $R_2$ are as hereinabove defined,

(c) hydrolyzing the latter amidoamine with a base or a protic source to obtain diamine having the formula $NH_2—CH(Ar)—CH_2—NH—CH_2CH_2—OR_1$ where $R_1$ and Ar are as above defined.

(d) reacting the resultant diamine with carbon disulfide at a temperature of from −10° to 40°C to obtain a dithiocarbamate,

(e) heating the latter dithiocarbamate at a temperature of from 80° to 150°C for from 2 to 20 hours to produce a thione having the formula:

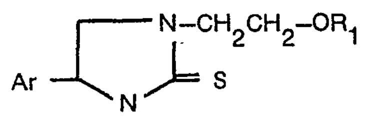

(f) further reacting the thione with an acid having the formula HA to provide a salt of tetramisole having the formula:

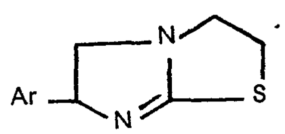 HA

where Ar is phenyl and A is an anion of a pharmaceutically acceptable acid, and

(g) if desired thereafter, neutralizing said tetramisole salt to obtain tetramisole *per se.*

2. A process according to Claim 1, wherein the reaction of step (a) is carried out at from 0° to 150°C.

3. A process according to Claim 1 or Claim 2, wherein the reaction of step (a) is carried out in an inert organic solvent.

4. A process according to Claim 3, wherein the organic solvent is selected from the group consisting of a saturated aliphatic hydrocarbon having up to 12 carbon atoms, a cycloaliphatic hydrocarbon and alkyl-substituted cycloaliphatic hydrocarbon, mononuclear aryl and alkyl- and nitro-substituted mono- and polynuclear aryl hydrocarbon, and mixtures thereof.

5. A process according to any preceding claim, wherein the alkoxyethylamine ranges from stoichiometric up to 15 times the molar quantity of the arylvinyl oxide.

## Revendications

1. Procédé de préparation du tétramisole caractérisé en ce qu'il comprend les stades de:

a) réaction d'un oxyde d'arylvinyle répondant à la formule:

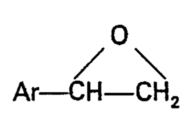

avec une alcoxyéthylamine répondant à la formule $R_1—O—(CH_2)_2—NH_2$ pour obtenir une N-(arylhydroxyalkyl)alcoxyéthylamine répondant à la formule: $Ar—CH(OH)—CH_2—NH—CH_2CH_2—OR_1$, dans lesquelles Ar représente le radical phényle et $R_1$ représente un radical alkyle en $C_1—C_6$,

b) réaction de cette dernière alcoxyéthylamine N-substituée en présence d'un acide minéral avec un nitrile répondant à la formule $R_2C{\equiv}N$, dans laquelle $R_2$ représente un atome d'hydrogène ou un radical alkyle ou aryle, pour obtenir une amidoamine répondant à la formule: $R_2—CO—NH—CH(Ar)—CH_2—NH—CH_2CH_2—OR_1$, dans laquelle Ar, $R_1$ et $R_2$ ont la même définition que ci-dessus,

c) hydrolyse de cette dernière amidoamine avec une base ou une source de protons pour obtenir une diamine répondant à la formule: $NH_2—CH(Ar)—CH_2—NH—CH_2CH_2—OR_1$ dans laquelle $R_1$ et Ar ont la même définition que ci-dessus,

d) réaction de la diamine obtenue avec du disulfure de carbone à une température de −10 à 40°C pour obtenir un dithiocarbamate,

e) chauffage de ce dernier dithiocarbamate à une température de 80 à 150°C pendant 2 à 20 heures pour produire une thione répondant à la formule:

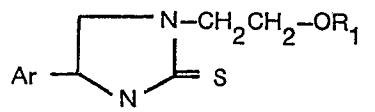

f) réaction de la thione avec un acide de formule HA pour fournir un sel de tétramisole de formule:

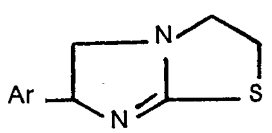

HA

dans laquelle Ar représente le radical phényle et A est un anion d'un acide pharmaceutiquement acceptable, et

g) si on le désire, neutralisation dudit sel de tétramisole pour obtenir le tétramisole lui-même,

2. Procédé selon la revendication 1, caractérisé en ce qu'on effectue le stade (a) entre 0 et 150°C.

3. Procédé selon la revendication 1 ou 2, caractérisé en ce qu'on effectue le stade de réaction (a) dans un solvant organique inerte.

4. Procédé selon la revendication 3, caractérisé en ce qu'on choisit le solvant organique parmi un hydrocarbure aliphatique saturé ayant jusqu'à 12 atomes de carbone, un hydrocarbure cycloaliphatique et un hydrocarbure cycloaliphatique alkyl-substitué, un hydrocarbure arylique mononucléaire ou polynucléaire alkyl-substitué et nitro-substitué, et leurs mélanges.

5. Procédé selon l'une quelconque des revendications précédentes, caractérisé en ce que la quantité d'alcoxyéthylamine est comprise entre la stoechiométrie et 15 fois la quantité molaire de l'oxyde d'arylvinyle.

**Patentansprüche**

1. Verfahren zur Herstellung von Tetramisol, gekennzeichnet durch die folgenden Stufen:

(a) Umsetzung eines Arylvinyloxids der Formel:

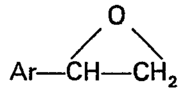

mit einem Alkoxyäthylamin der Formel: $R_1—O—(CH_2)_2—NH_2$ unter Erzeugung von N-(Arylhydroxyalkyl)alkoxyäthylamin der Formel: $Ar—CH(OH)—CH_2—NH—CH_2CH_2—OR_1$, wobei Ar für Phenyl steht und wobei $R_1$ für eine $(C_1—C_6)$-Alkylgruppe steht;

(b) Umsetzung des letzteren N-substituierten Alkoxyäthylamins in Gegenwart einer Mineralsäure mit einem Nitril der Formel $R_2C{\equiv}N$, wobei $R_2$ für Wasserstoff, Alkyl oder Aryl steht, unter Gewinnung eines Amidoamins der Formel: $R_2—CO—NH—CH(Ar)—CH_2—NH—CH_2CH_2—OR_1$, wobei Ar, $R_1$ und $R_2$ die oben angegebene Bedeutung haben;

(c) Hydrolyse des erhaltenen Amidoamins mit einer Base oder einer Protonenquelle unter Gewinnung eines Diamins der Formel $NH_2—CH(Ar)—CH_2—NH—CH_2CH_2—OR_1$, wobei $R_1$ and Ar die oben angegebene Bedeutung haben;

(d) Umsetzung des erhaltenen Diamins mit Schwefelkohlenstoff bei einer Temperatur von −10 bis 40°C unter Gewinnung eines Dithiocarbamats;

(e) Erhitzen des letzteren Dithiocarbamats bei einer Temperatur von 80 bis 150°C während 2 bis 20 Stunden unter Gewinnung eines Thions der Formel:

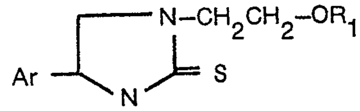

;

(f) Umsetzung des Thions mit einer Säure der Formel HA unter Gewinnung eines Salzes des Tetramisols der Formel:

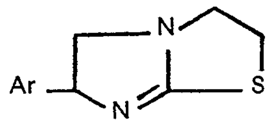

HA

wobei Ar für Phenyl steht und wobei A ein Anion einer pharmazeutisch akzeptablen Säure bedeutet; und

(g) gegebenenfalls Neutralisation des Tetramisolsalzes unter Gewinnung des freien Tetramisols.

2. Verfahren nach Anspruch 1, dadurch gekennzeichnet, daß man die Reaktionsstufe (a) bei 0 bis 150°C durchführt.

3. Verfahren nach einem der Ansprüche 1 oder 2, dadurch gekennzeichnet, daß man die Reaktion der Stufe (a) in einem inerten organischen Lösungsmittel durchführt.

4. Verfahren nach Anspruch 3, dadurch gekennzeichnet, daß man das organische Lösungsmittel auswählt aus der Gruppe der gesättigten aliphatischen Kohlenwasserstoffe mit bis zu 12 Kohlenstoffatomen, der cycloaliphatischen Kohlenwasserstoffe und der Alkyl-substituierten cycloaliphatischen Kohlenwasserstoffe, der einkernigen Arylkohlenwasserstoffe oder der Alkyl-substituierten oder Nitrosubstituierten, einkernigen oder mehrkernigen Arylkohlenwasserstoffe oder Mischungen derselben.

5. Verfahren nach einem der vorhergehenden Ansprüche, dadurch gekennzeichnet, daß die Menge des Alkoxyäthylamins im Bereich von der stöchiometrischen Menge bis zum 15fachen der molaren Menge, bezogen auf das Arylvinyloxid, liegt.